# EUROPEAN PATENT APPLICATION

(11) **EP 3 549 561 A1**
(43) Date of publication of application: **09.10.2019**
(21) Application number: 17875700.1
(22) Date of filing: 29.11.2017
(51) Int. Cl.: A61F 5/02

(54) **SUPPORT BAND**

(30) Priority: 30.11.2016 JP 2016232975
(71) Applicant: Poji Co., Ltd., Miki-shi, Hyogo 673-1122 (JP)
(72) Inventor: ISHIDA, Yoshinobu, Toyonaka-shi Osaka 560-0083 (JP)
(74) Representative: Zanoli, Enrico
(86) International application number: PCT/JP2017/042852
(87) International publication number: WO 2018/101345

(57) **Abstract**

A support band provided with a band body which has an intermediate strip part and hook-and-loop fastener parts connected to both ends in a longitudinal direction of the intermediate strip part,
wherein the hook-and-loop fastener parts each have a hook-shaped fiber surface part provided on one surface thereof and a loop-shaped fiber surface part that is provided on the other surface opposite to the one surface and is capable of bonding to the hook-shaped fiber surface part by surface contact,
each of the hook-shaped fiber surface parts and the loop-shaped fiber surface parts is continuously or intermittently provided so as to extend from one end part to the other end part in a longitudinal direction of each of the hook-and-loop fastener parts, and
the band body has a total length allowing the band body to be helically wrapped around a user from the lower abdomen, past the lower back, toward the chest.

## Description

### TECHNICAL FIELD

The present invention relates to a support band, and more particularly to a support band for supporting knees, lower back, or the like.

### BACKGROUND ART

During long hours of work in a seated position, such as during clerical job in an office, keeping a correct posture with one's chest out and back straight leads to a reduction in burden on the lower back. However, it is hard to keep the correct posture for a long time, and if not careful, a person is likely to bend forward from the waist unintentionally. Therefore, the number of people having lower back problems due to a long-time sitting position is increasing.

To reduce the burden on the lower back due to the sitting position as described above, a back support device disclosed in Patent Document 1 has been proposed, for example. The back support device is provided with upper and lower elastic straps, and a pair of loop-shaped elastic knee straps provided on both ends of the upper and lower elastic straps. The intermediate parts of the upper and lower elastic straps in the lengthwise direction are integrated, and a back support adjusting mechanism (hook-and-loop fastener) that can adjust the length is provided on the intermediate parts.

The back support device is worn such that the intermediate part is placed on the lower back of a person in a seated position, the pair of elastic knee straps is pulled to the front, and the loops thereof are fitted to the knees. The pair of elastic knee straps can also be connected to each other with a buckle member, whereby the knees can be kept together.

When the back support device is worn, the lower back of the user in a seated position is pushed to the front by the intermediate part, whereby the user can keep his/her back straight spontaneously. Thus, the burden on the lower back can be reduced.

Patent Document 2 discloses a waist supporter used for preventing or alleviating lower back pain. The waist supporter is provided with a stretchable belt body having a belt lower part and a belt upper part which are parallel to each other, the belt lower part and the belt upper part being connected at central parts in the longitudinal direction. The waist supporter is also provided with hook-and-loop fasteners composed of hook-shaped fiber surface parts and loop-shaped fiber surface parts on both ends of the belt lower part and on both ends of the belt upper part.

According to the waist supporter described above, the belt body is entirely wrapped around the abdominal region and fixed in such a way that the central part of the belt body is placed on the lower back region of a user, the belt lower part and the belt upper part are pulled to the abdominal region, and the hook-shaped fiber surface parts and the loop-shaped fiber surface parts of the hook-and-loop fasteners are overlapped and bonded to each other by surface contact. Thus, the waist supporter can alleviate or prevent lower back pain, while efficiently raising the abdominal pressure.

### CITATION LIST

### PATENT DOCUMENT

Patent Document 1: Japanese Patent No. 5319680
Patent Document 2: Japanese Unexamined Patent Publication No. 2015-139605

### SUMMARY OF THE INVENTION

### PROBLEM TO BE SOLVED BY THE INVENTION

The back support device disclosed in Patent Document 1 can be adjusted in length by the back support adjusting mechanism, and therefore, can address the difference in physical size of users, that is, can be adjusted to some degree according to the distance from the lower back to the knees of the user in a seated position. However, the adjustable range is narrow. Therefore, it is difficult to adjust the length to be suitable for both a tall person (190 cm tall, for example) and a short person (140 cm tall, for example). In addition, the back support device has a complex structure and high manufacturing cost, and thus imposes heavy economic burdens on users.

The waist supporter which is entirely wrapped around the abdominal region of a wearer as disclosed in Patent Document 2 cannot exhibit its effect if it is loosely wrapped around the abdominal region, and therefore, it needs to be wrapped with a certain degree of tightening force. Therefore, the abdominal region of the wearer is pressed by the waist supporter, and internal organs such as stomach or intestine are also pressed. Therefore, it is hard for the wearer to take a meal with the waist supporter on. Thus, the wearer has to take the waist supporter off when taking a meal, and even after the meal, the wearer has to refrain from wearing the waist supporter until a certain amount of time has elapsed.

Further, wearing of the waist support that presses the abdominal region for a long time may have various adverse effects on the human body. For example, breathing with a lower part of the lung is impaired, and burdens on the upper part of the lung are increased, which may cause respiratory problems. Another example is such that the internal organs such as stomach, kidney, or intestine are pressed down to cause splanchnoptosia, which may cause digestion difficulties or constipation. Still another example is such that the vein carrying blood in the lower part of the body to the heart is compressed to cause cardiovascular problems.

The conventional waist supporter described above presses and fixes the lower back region and the abdominal region, and limits the movement of muscles of the lower back region and the abdominal region to reduce the use of muscles, thereby alleviating pain. Examples of undesirable effects of the conventional waist supporter include: the wearer feeling tightness because the abdominal region is pressed; an occurrence of constriction of blood flow due to a long-time use; and an occurrence of muscle deterioration due to the limitation in the movement of muscles, and an increase in pain when the wearer takes the supporter off.

Further, muscle contraction occurs in the region having lower back pain (for example, acute low back pain), which may cause distortion of the body. However, the structure of pressing and fixing the lower back region and the abdominal region cannot eliminate the distortion of the body.

In addition, female clerks may have menstrual cramps in addition to lower back pain. In general, menstrual cramps can be alleviated by warming the abdominal region and the lower back region. For this reason, heating body warmers for alleviating menstrual cramps are commercially available. Such a heating body warmer has an adhesive sheet so that it can be attached on underwear or clothes, and continuously produces heat at about 40°C at which low temperature injury may not occur. However, the heating body warmer is relatively expensive, and costs too much for a user who uses the heating body warmer frequently.

The present invention is configured in view of the above problems, and an object of the present invention is to provide a support band which can be adapted to a variety of users having different physical sizes, correct the posture of a person in a seated position and reduce burdens on the lower back without pressing and fixing the lower back region and the abdominal region, is applicable to support other parts of the body, and can reduce economical burdens on users.

### MEANS FOR SOLVING THE PROBLEMS

The present invention provides a support band comprising a band body which has an intermediate strip part and hook-and-loop fastener parts connected to both ends in a longitudinal direction of the intermediate strip part,
wherein the hook-and-loop fastener parts each have a hook-shaped fiber surface part provided on one surface and a loop-shaped fiber surface part that is provided on the other surface opposite to the one surface and is capable of bonding to the hook-shaped fiber surface part by surface contact,
each of the hook-shaped fiber surface parts and the loop-shaped fiber surface parts is continuously or intermittently provided so as to extend from one end part to the other end part in a longitudinal direction of each of the fastener parts, and
the band body has a total length allowing the band body to be helically wrapped around a user from the lower abdomen, past the lower back, toward the chest.

According to another aspect, the present invention provides a support band comprising a band body which has an intermediate strip part and hook-and-loop fastener parts connected to both ends in a longitudinal direction of the intermediate strip part,
wherein the hook-and-loop fastener parts each have a hook-shaped fiber surface part provided on one surface and a loop-shaped fiber surface part that is provided on the other surface opposite to the one surface and is capable of bonding to the hook-shaped fiber surface part by surface contact,
each of the hook-shaped fiber surface parts and the loop-shaped fiber surface parts are continuously or intermittently provided so as to extend from one end part to the other end part in a longitudinal direction of each of the fastener parts, and
the hook-and-loop fastener parts each have a length allowing the hook-and-loop fastener part to be wrapped around the leg of a user at least once.

### EFFECTS OF THE INVENTION

The band body of the support band according to the present invention has a simple structure provided with the hook-and-loop fastener parts on both ends in the longitudinal direction of the intermediate strip part. Each of the hook-and-loop fastener parts is provided with the hook-shaped fiber surface part and the loop-shaped fiber surface part which are formed continuously or intermittently in the longitudinal direction on the front surface and the back surface, respectively.

Further, in the support band, the band body has a total length allowing the band body to be helically wrapped around the user from the lower abdomen, past the lower back, toward the chest, or the hook-and-loop fastener parts each have a length allowing the hook-and-loop fastener part to be wrapped around the leg of the user at least once.

With the configuration described above, the support band according to the present invention can be fitted to the body in various ways of wearing despite the simple configuration, and thus, can be used as a supporter for various body parts such as shoulder, chest, abdomen, lower back (lumbar), arm, pelvis, hip joint, leg, knee, and ankle. Specifically, a single support band can be used in various ways and can reliably support various body parts, and thus, the need to prepare supporters specialized for respective body parts is eliminated. In addition, the support band according to the present invention has a simple structure, which leads to reduction in manufacturing cost. Accordingly, economical burdens on the user can be reduced by using the support band according to the present invention.

The hook-shaped fiber surface part and the loop-shaped fiber surface part of each hook-and-loop fastener part are provided to continuously or intermittently extend from one end part to the other end part, whereby the hook-shaped fiber surface part and the loop-shaped fiber surface part of each hook-and-loop fastener part can be reliably bonded to each other by surface contact, regardless of the width of the knee of the user. Thus, the support band according to the present invention is usable for any users having different physical sizes. In addition, when the support band is entirely wrapped around the knee or pelvis of the user to support the knee or lower back, the hook-shaped fiber surface part of one of the hook-and-loop fastener parts and the loop-shaped fiber surface part of the other hook-and-loop fastener parts can be reliably bonded by surface contact and fixed. Thus, the support band according to the present invention can be used, regardless of physical sizes of users.

### BRIEF DESCRIPTION OF THE DRAWINGS

FIG. 1 is a plan view illustrating a support band according to a first embodiment of the present invention.
FIG. 2 is a bottom view of the support band illustrated in FIG. 1.
FIG. 3(A) is an explanatory view illustrating a bonding state of a pair of hook-and-loop fastener parts of the support band according to the first embodiment, and FIG. 3(B) is an explanatory view illustrating a state in which the bonding state is released.
FIG. 4 illustrates a first wearing example for correcting misalignment of spine using the support band according to the first embodiment, wherein (A) is a view seen from front, (B) is a view seen from the right side, and (C) is a view seen from back.
FIG. 5 illustrates the first wearing example for correcting a stance before golf swing using the support band according to the first embodiment, wherein (A) is a view seen from front, (B) is a view seen from the right side, and (C) is a view seen from back.
FIG. 6 illustrates a second wearing example for correcting a spine into an S-shaped curve using the support band according to the first embodiment, wherein (A) is a view seen from front, and (B) is a view seen from the left side.
FIG. 7 illustrates a third wearing example for correcting a spine into an S-shaped curve using the support band according to the first embodiment, wherein (A) is a view seen from front, and (B) is a view seen from the left side.
FIG. 8 is an explanatory view illustrating a fourth wearing example for correcting a spine into an S-shaped curve using the support band according to the first embodiment.
FIG. 9 is an explanatory view illustrating a fifth wearing example for supporting a knee using the support band according to the first embodiment.
FIG. 10 is an explanatory view illustrating a sixth wearing example for supporting an ankle using the support band according to the first embodiment.
FIG. 11 is an explanatory view illustrating a seventh wearing example for supporting an arm using the support band according to the first embodiment.
FIG. 12 illustrates an eighth wearing example for supporting the pelvis using the support band according to the first embodiment, wherein (A) is a view seen from front, and (B) is a view seen from back.
FIG. 13 illustrates a ninth wearing example for supporting the chest using the support band according to the first embodiment, wherein (A) is a view seen from front, and (B) is a view seen from back.
FIG. 14 illustrates a tenth wearing example for supporting a hip joint using the support band according to the first embodiment, wherein (A) is a view seen from front, (B) is a view seen from the left side, and (C) is a view seen from back.
FIG. 15 is a plan view illustrating a support band according to a second embodiment of the present invention.
FIG. 16(A) is a left side view illustrating a bag member of the support band according to the second embodiment, (B) is a right side view thereof, and (C) is a left sectional view thereof.
FIG. 17 is an explanatory view illustrating a support band according to a third embodiment of the present invention.
FIG. 18 illustrates an eleventh wearing example for warming or cooling an abdominal region and lower back region using the support band according to the second or third embodiment, wherein (A) is a view seen from the right side, and (B) is a view seen from the left side.
FIG. 19 illustrates a twelfth wearing example for warming or cooling a shoulder using the support band according to the second or third embodiment, wherein (A) is a view seen from front, (B) is a view seen from the right side, and (C) is a view seen from back.
FIG. 20 illustrates a thirteenth wearing example for warming or cooling both shoulders using the support band according to the second or third embodiment, wherein (A) is a view seen from front, and (B) is a view seen from back.

### MODES FOR CARRYING OUT THE INVENTION

The support band according to the present invention is provided with a band body which has an intermediate strip part and hook-and-loop fastener parts connected to both ends in the longitudinal direction of the intermediate strip part,
wherein the hook-and-loop fastener parts each have a hook-shaped fiber surface part provided on one surface and a loop-shaped fiber surface part that is provided on the other surface opposite to the one surface and is capable of bonding to the hook-shaped fiber surface part by surface contact, and
each of the hook-shaped fiber surface parts and the loop-shaped fiber surface parts is continuously or intermittently provided so as to extend from one end part to the other end part in a longitudinal direction of each of the fastener parts.

The support band is also configured such that: (A) the band body has a total length allowing the band body to be helically wrapped around a user from the lower abdomen, past the lower back, toward the chest of the user; and (B) the hook-and-loop fastener parts each have a length allowing the hook-and-loop fastener part to be wrapped around the leg of the user at least once.

The support band having the configuration (A) can be used as described in the following (I) or (II).
(I) Two support bands are wrapped around the user in a helical manner in reverse directions from the lower abdomen, past the lower back, toward the chest of the user, and the hook-and-loop fastener parts on both ends of the respective support bands are bonded to each other by surface contact on the lower abdomen and the chest. In this case, the band body of each support band has a total length allowing the band body to be wrapped around the user in at least a single helical manner from the lower abdomen, past the lower back, toward the chest of the user. In order to use the support bands for various users having different physical sizes, the band body may have a length of about 80 to 110 cm, for example.
(II) With the intermediate part of a single support band in the longitudinal direction being placed on the lower abdomen of the user, the support band is wrapped around the user in a double helical manner in reverse directions from the lower abdomen, past the lower back, toward the chest, and the hook-and-loop fastener parts on both ends of the support band are bonded to each other by surface contact on the chest. The total length of the support band in this case is about twice (about 160 to 220 cm) the total length of the support band used in (I) described above.

According to the wearing examples in (I) and (II), the broadest muscle of the back of the user can be supported without pressing the lower back, whereby the excessive muscle tension can be relieved to alleviate lower back pain, and the posture can be adjusted (corrected) upright. Therefore, these wearing examples are particularly effective for appropriately supporting the body of the user having acute low back pain. Further, the support band having the length enabling the wearing examples described above can be used in various ways. For example, the support band is wrapped twice around the waist for supporting the lower back region, or when the user breaks his/her arm, the user wears the support band around the neck and supports the arm in a sling as a first-aid treatment.

The support band having the configuration (B) can be used such that one of the hook-and-loop fastener parts of the support band is wrapped around the knee on the shin side and fixed, the support band is wrapped around the leg toward the thigh, and the other hook-and-loop fastener part is fixed on the thigh. Thus, the knee can be firmly supported by the support band. Further, the hook-and-loop fastener part has a length allowing the hook-and-loop fastener part to be wrapped around the leg once, and thus, the support band can be used in various ways. For example, the support band is wrapped around the ankle, the elbow, or the like for supporting the same, or the support band is wrapped around the broken arm for supporting the same as a first-aid treatment. In this case, the hook-and-loop fastener part can be set to have a length of bout 50 to 70 cm in consideration of the size of legs of various users.

The support band thus configured may have configurations described below, and these configurations may be combined, as appropriate.
(1) The intermediate strip part may have an elongation rate greater than an elongation rate of the hook-and-loop fastener parts.
   With this configuration, even if the intermediate strip part is wrapped around the abdominal region of the user, pressing force on the abdominal region can be reduced due to stretching properties of the intermediate strip part.
(2) The intermediate part may have an elongation rate of at least 1.1.
   With this configuration, the intermediate strip part may have stretching properties by which the intermediate strip part can sufficiently follow the movement of the user. Therefore, the support band fitted to the user easily maintains a tight state without loosening, with the result that the surface-contact bonding between the hook-shaped fiber surface part and the loop-shaped fiber surface part is less likely to get loose.
(3) The support band may further have a bag member attached to the band body in a detachable manner,
   wherein the bag member may have a bag body, a heat buffer material accommodated in the bag body, a pocket part into which a heating member or a heat absorbing member is accommodated, the pocket part being provided on an outer surface side of the bag body, and a loop part which is provided on the outer surface side of the bag body and into which the band body is inserted.
   With this configuration, when the support band is wrapped around the abdomen of the user with a commercially available disposable body warmer being put into the pocket part, the lower back or the abdomen can be warmed while the lower back is supported. If two bag members are attached to the band body, the lower back and the abdomen of the user can be simultaneously warmed.
   The support band provided with the bag member and the band body according to the present invention is suitable for commute and outdoor work in a cold season, for example. The support band is also preferable for alleviating menstrual cramps of female clerks who may work for many hours in a seated position. In such a case, due to the heat buffer material being provided between the body of the user and the disposable body warmer, heat of the disposable body warmer transmitted to the user is alleviated, and thus, an occurrence of low temperature injury (burns) can be prevented. In addition, the configuration described above allows the use of relatively inexpensive disposable body warmers, and thus can prevent frequent use of expensive heating body warmers for alleviating menstrual cramps, thereby reducing economical burdens on the user.
   If the user needs to cool the injured lower back, he/she may wrap the support band around the abdomen with a commercially available refrigerant being put into the pocket part. Thus, the lower back can be simultaneously supported and cooled. In this case, due to the heat buffer material being provided between the refrigerant and the band body, the refrigerant can be arranged closer to the user, and thus, the lower back can be efficiently cooled.
(4) The heat buffer material may be formed from resin foam beads.

With this configuration, the bag member is easily deformed into a shape fitted to the body of the user, whereby the user can use the support band by wrapping the same around the body without any feeling of strangeness. Further, due to the configuration in which the heat buffer material is formed from resin foam beads, if the resin foam beads are collected to the central part of the bag body to increase the thickness, the heat alleviation is increased, and if the resin foam beads are collected to the outer peripheral part of the bag body to reduce the thickness at the central part, the heat alleviation can be reduced. In this way, the temperature of heat transmitted to the user can be easily adjusted.

Hereinafter, support bands according to embodiments of the present invention will be described in detail with reference to the accompanying drawings.

### (First embodiment)

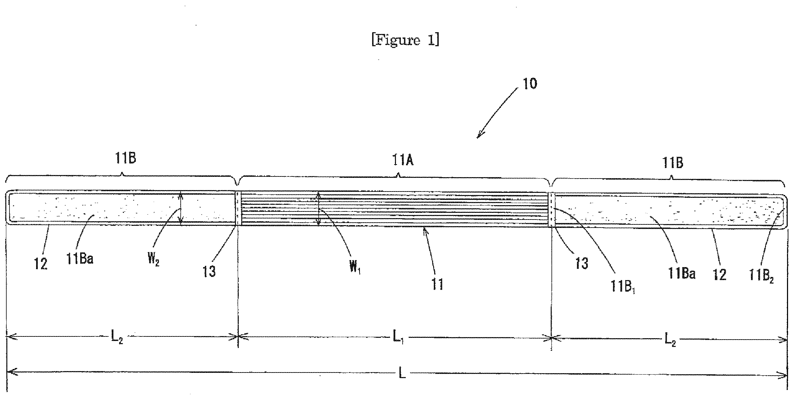

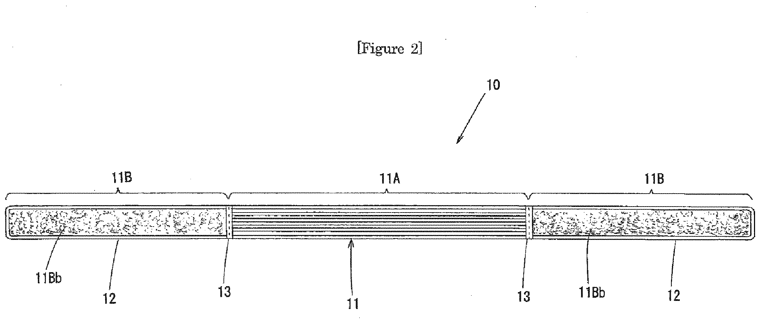

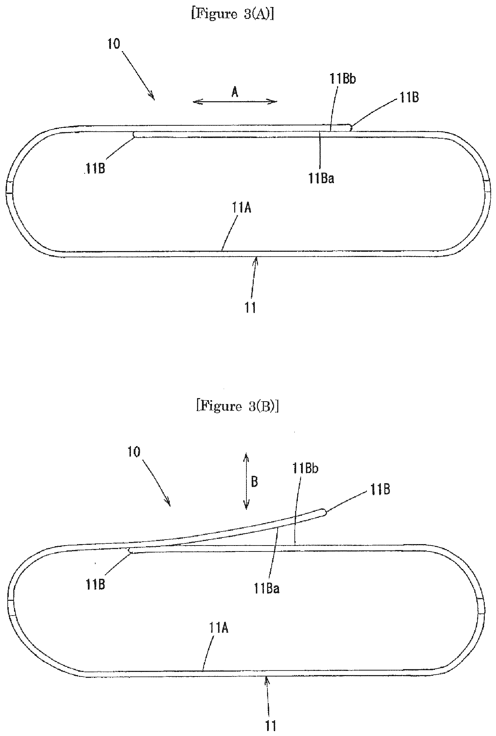

FIG. 1 is a plan view illustrating a support band according to a first embodiment of the present invention, and FIG. 2 is a bottom view of the support band illustrated in FIG. 1. FIG. 3(A) is an explanatory view illustrating a bonding state of a pair of hook-and-loop fastener parts of the support band according to the first embodiment, and FIG. 3(B) is an explanatory view illustrating a state in which the bonding state in FIG. 3(A) between the pair of hook-and-loop fastener parts of the support band is released.

A support band 1 according to the first embodiment is provided with a band body which has a stretchable part 11A which is stretchable and hook-and-loop fastener parts 11B connected to both ends of the stretchable part 11A in the longitudinal direction.

The stretchable part 11A is formed from a rubber band having a predetermined width W and a length L₁, and has an elongation rate of at least 1.1. The elongation rate is obtained by measuring the maximum length of the rubber band when the rubber band is broken after being pulled from both sides in the longitudinal direction, and calculating a ratio of the maximum length to the original length L₁. In the present invention, the elongation rate of the stretchable part 11A is preferably at least 1.1, and from the viewpoint of practical use, it is 1.1 to 1.3.

Each of the hook-and-loop fastener parts 11B is formed from a cloth material having a width W₂ substantially equal to the width W₁ of the stretchable part 11A, and is provided with a hook-shaped fiber surface part 11Ba formed on one surface thereof and a loop-shaped fiber surface part 11Bb which is formed on the other surface opposite to the one surface and which can be bonded by surface contact to the hook-shaped fiber surface part 11Ba.

Further, the hook-shaped fiber surface part 11Ba and the loop-shaped fiber surface part 11Bb are continuously provided from one end part 11B₁ to the other end part 11B₂ of the hook-and-loop fastener part 11B. In the first embodiment, a trimming tape 12 both for decoration and stopping frays of the outer periphery of the hook-and-loop fastener part 11B is sewn on the hook-and-loop fastener part 11B.

Cloth materials 13 are sewn along the borders between the stretchable part 11A and the hook-and-loop fastener parts 11B, whereby the pair of hook-and-loop fastener parts 11B is connected to both ends of the stretchable part 11A in the longitudinal direction.

The total length L of the band body 11 is not particularly limited. However, the band body 11 may preferably have a length allowing the band body 11 to be applied to a user having a large physical size in various ways of wearing described later, and preferably have a length of about 160 to 220 cm from the viewpoint of practical use. Further, various sizes may be prepared such as 160 cm for S size, 190 cm for M size, and 220 cm for L size.

The length L₂ of each of the hook-and-loop fastener parts 11B is not particularly limited. However, each of the hook-and-loop fastener parts 11B preferably has a length allowing the hook-and-loop fastener part 11B to be wrapped around the knee (calf or thigh near the knee) of the user with a large physical size at least once, and preferably has a length of about 50 to 70 cm from the viewpoint of practical use. The length may be set for each size, such as 50 cm for S size, 60 cm for M size, and 70 cm for L size.

The length L₁ of the stretchable part 11A can be set to be 65 to 90 cm in consideration of the total length L of the band body 11 and the length L₂ of each hook-and-loop fastener part 11B. The width W₁ of the stretchable part 11A is not particularly limited, and can be set to be 6 to 12 cm, for example. Note that the width W₂ of each the hook-and-loop fastener part 11B may be larger or smaller than the width W₁ of the stretchable part 11A by about several centimeters.

Commercially available known products provided with a set of hook-shaped fiber surface part and loop-shaped fiber surface part, that is, known hook-and-loop fasteners such as Magic Tape (registered trademark), Velcro (registered trademark), Fastenano (registered trademark), and SNAPPING FABRICS disclosed in U.S. Patent No. 7,231,789, can be used for the hook-shaped fiber surface parts 11Ba and loop-shaped fiber surface parts 11Bb of the hook-and-loop fastener parts 11B.

In the Magic Tape and Velcro, a hook material and a loop material are separately provided. Therefore, when each of the hook-and-loop fastener parts 11B is manufactured using the Magic Tape or Velcro, the hook material (or loop material) is entirely sewn on one surface of a cloth base material, and then, the loop material (or hook material) is entirely sewn on the other surface of the cloth base material, for example.

The commercially available Fastenano and SNAPPING FABRICS are provided with the hook-shaped fiber surface part 11Ba formed on the entire one surface of a cloth base material and the loop-shaped fiber surface part 11Bb formed on the entire other surface of the cloth base material. Therefore, the Fastenano and SNAPPING FABRICS can be used as the hook-and-loop fastener parts 11B without providing any treatment. The first embodiment shows the hook-and-loop fastener parts 11B formed from SNAPPING FABRICS having an elongation rate of about 1.

As illustrated in FIG. 3(A), the hook-and-loop fastener parts 11B formed by using Fastenano or SNAPPING FABRICS are bonded to each other in such a way that the band body 11 is looped, and the hook-shaped fiber surface part 11Ba of one of the hook-and-loop fastener parts 11B is brought into surface contact with the loop-shaped fiber surface part 11Bb of the other hook-and-loop surface part 11B. The bonding force of the surface contact is strong relative to force in a direction (indicated by an arrow A) in which the pair of hook-and-loop fastener parts 11B is relatively stretched, but is weak relative to force in a direction (indicated by an arrow B) in which the pair of hook-and-loop fastener parts 11B is relatively peeled, as illustrated in FIG. 3(B).

Therefore, when the support band 10 according to the present invention is worn by the user, the hook-and-loop fastener parts 11B are less likely to be separated from each other, except for the case where the user intends to release the bonding state by surface contact between the hook-shaped fiber surface part 11Ba and the loop-shaped fiber surface part 11Bb.

Now, various methods (wearing examples) for using the support band 10 according to the present invention will be described.

### <First wearing example>

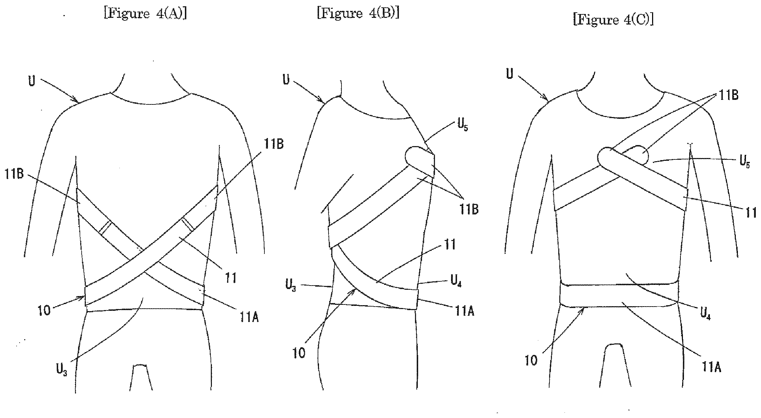

FIG. 4 illustrates a first wearing example for correcting misalignment of spine using the support band according to the first embodiment, wherein (A) is a view seen from front, (B) is a view seen from the right side, and (C) is a view seen from back.

For example, in the first wearing example, the stretchable part 11A is placed on the abdomen U₄ of the user U and stretched so that the pair of hook-and-loop fastener parts 11B cross near the lower back U₃ on the back, then, the pair of hook-and-loop fastener parts 11B is pulled toward the chest U5, and the hook-shaped fiber surface part 11Ba of one of the hook-and-loop fastener parts 11B and the loop-shaped fiber surface part 11Bb of the other hook-and-loop fastener part 11B are bonded by surface contact and fixed to each other in an X shape. Specifically, with intermediate part of the single support band in the longitudinal direction being placed on the lower abdomen of the user U, the support band is wrapped around the user from the lower abdomen, past the lower back, toward the chest, in a double helical manner in reverse directions, and the hook-and-loop fastener parts on both ends of the support band are bonded by surface contact to each other on the chest.

According to the first wearing example, the region from the lower back U₃ to the chest U₅ can be supported without giving pressure to the abdomen U₄ of the user U, and thus, misalignment of spine can be corrected, and muscle tension of the lower back can be relieved. Further, the body of the user U who has acute low back pain can be appropriately supported. Due to the band body 11 having a total length of at least 170 cm, the abovementioned method for using the support band is possible, and well applicable to a user having a large physical size.

More specifically described, in the state where the support band 10 is worn as illustrated in FIG. 4, the support band 10 is worn to form an X shape over a region from a part below the right anterior chest to the left anterior pelvis and over a region from a part below the left anterior chest to the right anterior pelvis of the user U, and the crossing portion of the X shape is positioned near the lumbar of the user U and can support the erector spinae muscles of the lower back U₃. Therefore, the muscle tension of the lower back U₃ can be relieved, and distortion between the left and right sides of the body can be resolved. Further, the region near the lower back U₃ is pressed to the front by the crossing portion of the X shape, and the chest U₅ is pulled to the back by both ends. Therefore, the spine of the user U is corrected into a normal S-shaped curve, and thus, the lower back pain can be alleviated or prevented.

In the wearing state illustrated in FIG. 4, the abdominal region of the user is not pressed when the user wears the support band. Therefore, various adverse effects on the human body caused by pressing the abdominal region, such as respiratory problems, digestion difficulties, and cardiovascular problems described above are less likely to occur. Further, even when the user takes a meal with the support band on, the user does not suffer from abdominal pain. Moreover, the user can do desk work in a seated position and bend slightly forward comfortably. Thus, the support band can be used safely and healthfully in daily life.

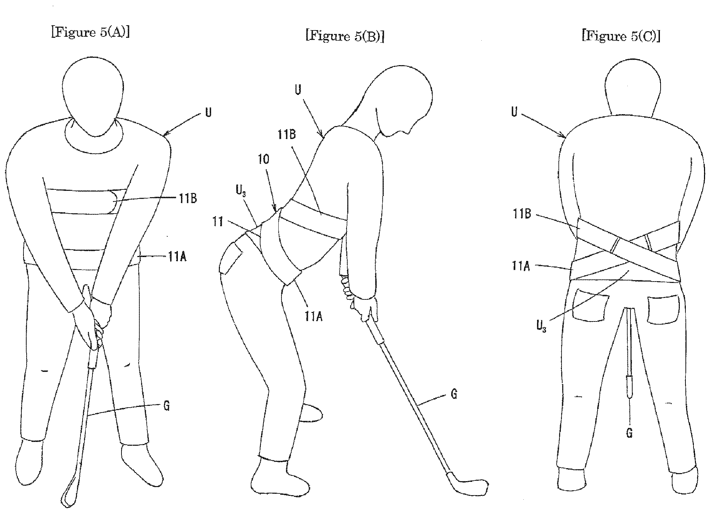

FIG. 5(A) illustrates the first wearing example for correcting a stance before golf swing using the support band according to the first embodiment, wherein (A) is a view seen from front, (B) is a view seen from the right side, and (C) is a view seen from back. As illustrated in FIG. 5, in the first wearing example, the support band is applicable to correct the posture so that the user U swings a golf club G with his/her back straight.

Further, for a person carrying a heavy load, a person working in a bent-forward posture, a person doing sports for hitting a ball with a twisting motion such as golf, tennis, or baseball, or the like, the body supporter 10 according to the present invention is useful for supporting the muscles of the lower back of the user to reduce burdens on the lower back and improving performance of muscles.

FIGS. 4 and 5 show an example where a single support band is wrapped around the body of the user in a double helical manner in reverse directions. However, in this wearing example, the single support band may be divided into two (not illustrated). In such a case, two support bands are wrapped around the body from the lower abdominal region, past the lower back, toward the chest of the user in a double helical manner in reverse directions, and the hook-and-loop fastener parts of the respective support bands are bonded by surface contact to each other on the lower abdominal region and the chest. In this case, the length of the support band is about 80 to 110 cm, for example.

### <Second wearing example>

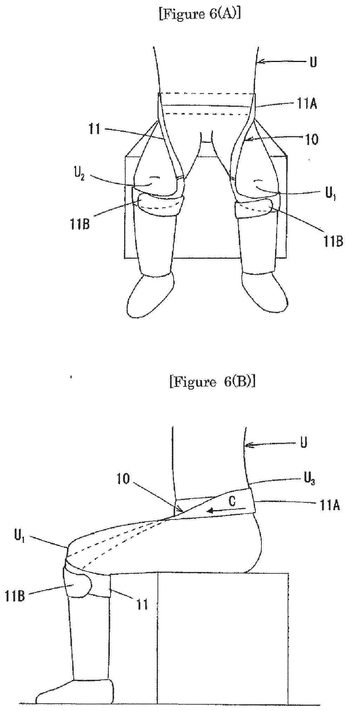

FIG. 6 illustrates a second wearing example for correcting the spine into an S-shaped curve using the support band according to the first embodiment, wherein (A) is a view seen from front, and (B) is a view seen from the left side.

In this case, for example, one of the hook-and-loop fastener parts 11B is wrapped around a region under the left knee U₁ of the user U seated in a chair, and the hook-shaped fiber surface part 11Ba on one surface and the loop-shaped fiber surface part 11Bb on the other surface are bonded by surface contact and fixed (see FIG. 3(A)). Then, the stretchable part 11A is pulled around the lower back toward the right knee U₂, the other hook-and-loop fastener part 11B is wrapped around a region under the right knee U₂, and the hook-shaped fiber surface part 11Ba on one surface and the loop-shaped fiber surface part 11Bb on the other surface are bonded by surface contact and fixed.

When the user wears the support band 10 as in the second wearing example, the lower back U₃ of the user U in a seated position is pressed to the front (in the direction of arrow C) by the stretchable part 11A. Thus, the user U can assume a posture with his/her back straight spontaneously, whereby burdens on the lower back U₃ are reduced while the user is continuously in the seated position for a long time. Because each of the hook-and-loop fastener parts has a length of at least 50 cm, the hook-and-loop fastener part 11B can be wrapped at least once around the large knee of the user U having a large physical size and fixed.

### <Third wearing example>

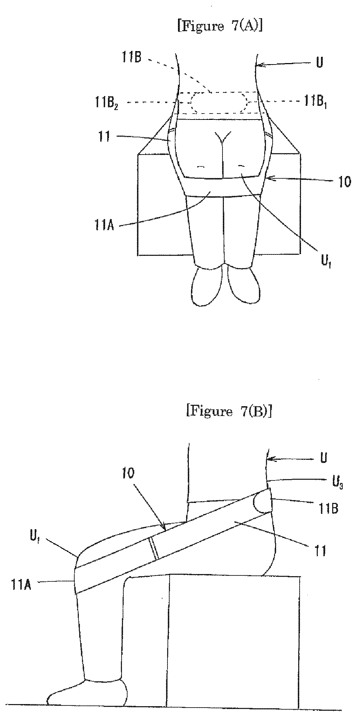

FIG. 7 illustrates a third wearing example for correcting the spine into an S-shaped curve using the support band according to the first embodiment, wherein (A) is a view seen from front, and (B) is a view seen from the left side.

In the third wearing example, the support band is worn in such a way that the hook-and-loop fastener parts 11B on both ends of the band body 11 are wrapped around the lower back U₃ of the user U seated in a chair with the stretchable part 11A of the band body 11 being placed on the left knee U₁ and the right knee U₂, and the one end part 11B1 and the other end part 11B2 are overlapped with each other and bonded to each other by surface contact.

The third wearing example is easier and requires less force than the second wearing example when the user wears the support band. Further, the user U spontaneously assumes a posture with knees together. Therefore, the third wearing example is suitable for a woman having relatively weak arm strength and caring about the posture with her knees apart.

### <Fourth wearing example>

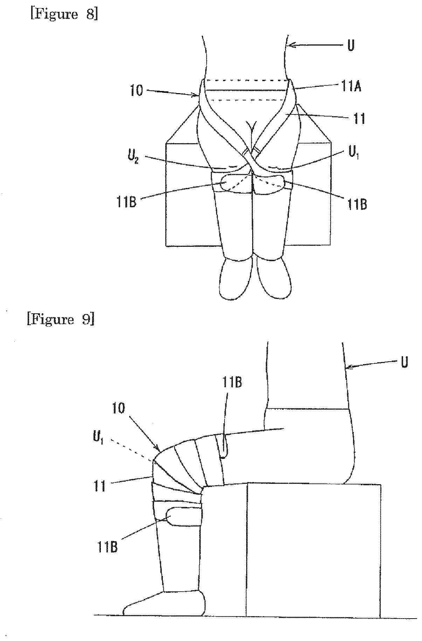

FIG. 8 is an explanatory view illustrating a fourth wearing example for correcting the spine into an S-shaped curve using the support band according to the first embodiment.

In the fourth wearing example, for example, one of the hook-and-loop fastener parts 11B is firstly wrapped around the region under the right knee U₂ of the user U seated in a chair, and the hook-shaped fiber surface part 11Ba on one surface and the loop-shaped fiber surface part 11Bb on the other surface are bonded to each other by surface contact and fixed (see FIG. 3(A)). Then, the stretchable part 11A is placed on the lower back on the left side and pulled to the left knee U₁, the other hook-and-loop fastener part 11B is wrapped around the region under the left knee U₁, and the hook-shaped fiber surface part 11Ba on one surface and the loop-shaped fiber surface part 11Bb on the other surface are bonded by surface contact and fixed.

When the user wears the support band 10 as in the fourth wearing example, the pair of hook-and-loop fastener parts 11B cross each other in the vicinity of knees and fixed to the regions under the left and right knees U₁ and U₂. Thus, the left and right knees U₁ and U₂ are brought close to each other, and the user U is spontaneously seated with his/her legs held together, whereby the burdens on the lower back U₃ are reduced. When the user wears the support band 10 in the manner described above in the seated position, the pair of hook-and-loop fastener parts 11B are stretched by the stretchable part 11A, and therefore, the surface-contact bonding between the hook-and-loop fastener parts 11B fixed at the regions under the left and right knees U₁ and U₂ is less likely to be accidentally released. Meanwhile, if the user peels the tip ends of the hook-and-loop fastener parts 11B, the surface-contact bonding is easily released, whereby the user U can quickly take off the support band 10 when the user U leaves his/her seat.

### <Fifth wearing example>

FIG. 9 is an explanatory view illustrating a fifth wearing example for supporting the knee using the support band according to the first embodiment.

The fifth wearing example shows a method for entirely supporting the knee of the user U with the support band 10. For example, the support band 10 is wrapped around the leg from the thigh above the knee toward the calf under the knee, and the hook-shaped fiber surface part 11Ba on one surface of one of the hook-and-loop fastener parts 11B at the terminal end and the loop-shaped fiber surface part 11Bb on the other surface thereof are bonded by surface contact to each other and fixed (see FIG. 3(A)). In this case, the surface-contact bonding of the hook-and-loop fastener part 11B is also less likely to be accidentally released, and the user can easily take off the support band 10.

### <Sixth wearing example>

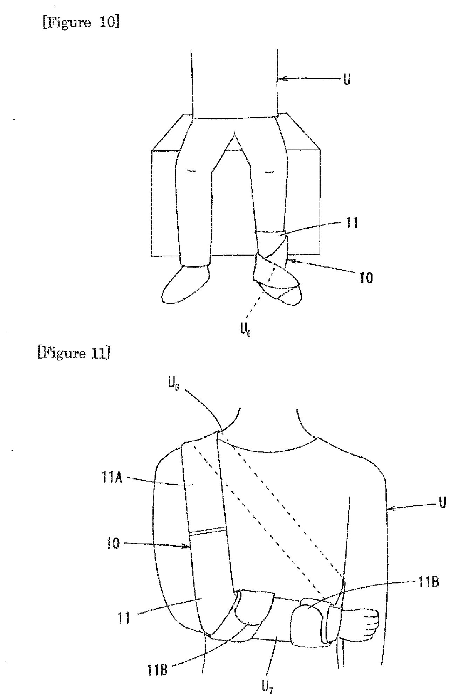

FIG. 10 is an explanatory view illustrating a sixth wearing example for supporting the ankle using the support band according to the first embodiment.

For example, when the user U twists (sprains) the ankle U₆ at home or away from home, the ankle U₆ of the user U can be protected by wrapping the support band 10 around the ankle U₆ as in the sixth wearing example as a first-aid treatment.

### <Seventh wearing example>

FIG. 11 is an explanatory view illustrating a seventh wearing example for supporting the arm using the support band according to the first embodiment.

For example, when the user injures (breaks) the arm U₇ at home or away from home, the hook-and-loop fastener parts 11B on both ends of the support band 10 are wrapped around the arm U₇ of the user U, and the stretchable part 11A are placed on the shoulder U₈ as in the seventh wearing example. With this, the arm U₇ can be supported in a sling as a first-aid treatment without placing any burdens. In such a case, the support band 10 may be wrapped around a splint applied on the arm U₇.

### <Eighth wearing example>

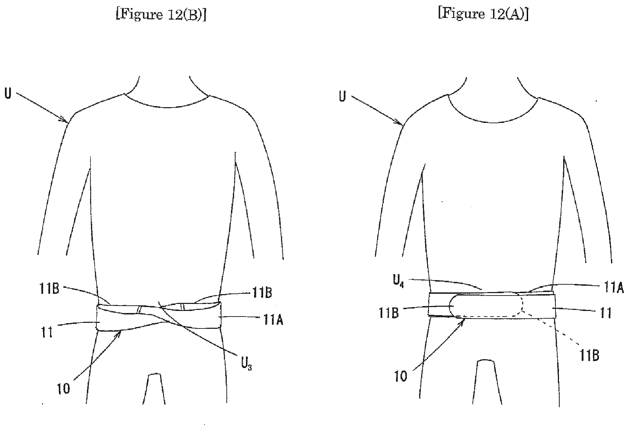

FIG. 12 illustrates an eighth wearing example for supporting the pelvis using the support band according to the first embodiment, wherein (A) is a view seen from front, and (B) is a view seen from back.

In the eighth wearing example, the support band 10 is wrapped around the pelvis with the intermediate part of the stretchable part 11A being placed on the abdomen U₄ of the user U, and the hook-shaped fiber surface part 11Ba of one of the hook-and-loop fastener parts 11B and the loop-shaped fiber surface part 11Bb of the other hook-and-loop fastener part 11B are bonded to each other by surface contact, for example.

With this, the region around the pelvis can also be supported by the support band 10 when the user U stands up and walks, and further, the user U can be seated with the support band 10 on. This wearing example is particularly suitable for reducing the lower back pain of the user U.

### <Ninth wearing example>

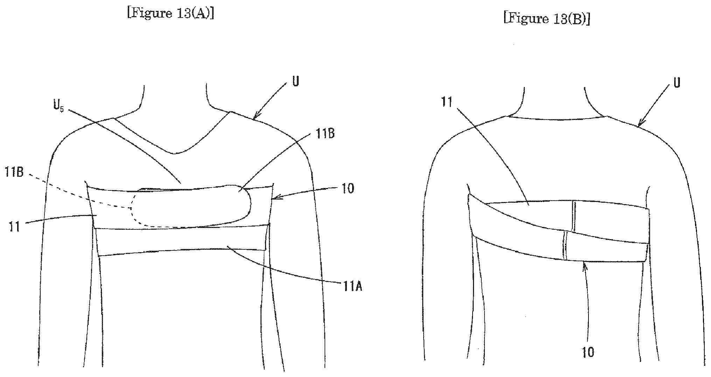

FIG. 13 illustrates a ninth wearing example for supporting the chest using the support band according to the first embodiment, wherein (A) is a view seen from front, and (B) is a view seen from back.

For example, when the user injures (breaks) a rib at home or away from home, the support band 10 is wrapped around the chest U₅ of the user U as in the ninth wearing example, whereby the rib can be protected as a first-aid treatment.

### <Tenth wearing example>

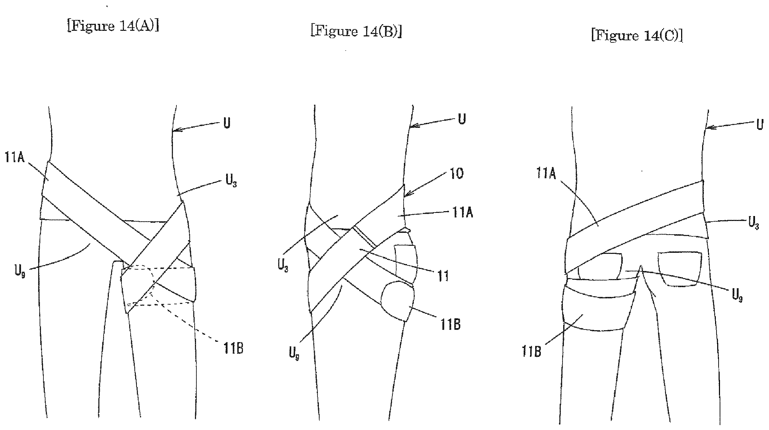

FIG. 14 illustrates a tenth wearing example for supporting the hip joint using the support band according to the first embodiment, wherein (A) is a view seen from front, (B) is a view seen from the left side, and (C) is a view seen from back.

For example, when the user injures the hip joint at home or away from home, the support band 10 is wrapped around a region from the base of the leg to the lower back U of the user U as in the tenth wearing example, whereby the hip joint can be protected as a first-aid treatment without placing any burdens.

### (Second and third embodiments)

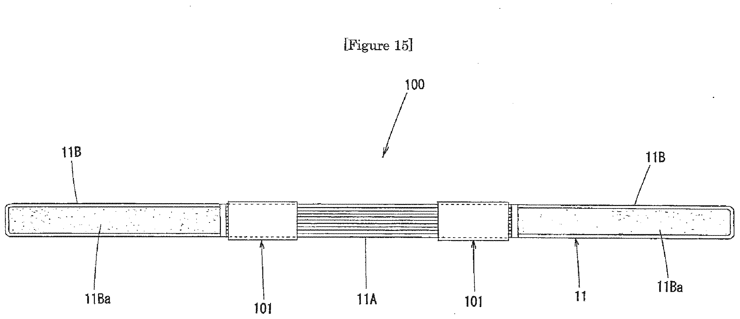

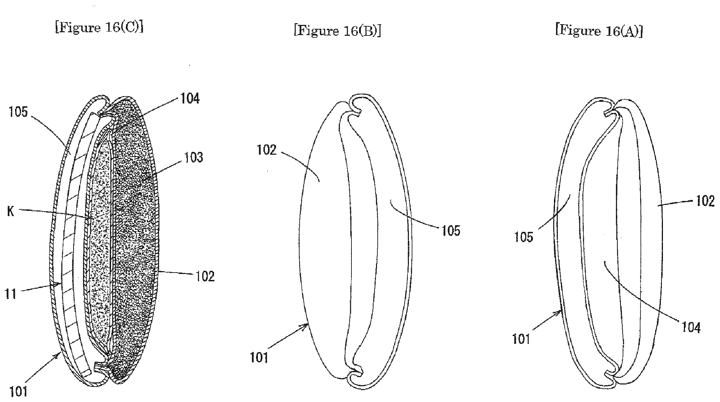

FIG. 15 is a plan view illustrating a support band according to a second embodiment of the present invention. FIG. 16(A) is a left side view illustrating a bag member of the support band according to the second embodiment, (B) is a right side view thereof, and (C) is a left sectional view thereof. In FIG. 15, the components same as the components in FIGS. 1 to 3 are identified by the same reference marks.

A support band 100 according to the second embodiment is provided with the band body 11 described in the first embodiment and a bag member 101 attached to the band body 11 in a detachable manner. The second embodiment shows an example where two bag members 101 are provided. However, the number of the bag members 101 is not particularly limited, and one or more than two bag members may be provided.

The bag member 101 has a bag body 102, a heat buffer material 103 accommodated in the bag body, a pocket part 104 for accommodating a heating body, the pocket part 104 being provided on an outer surface side of the bag body 102, and a loop part 105 which is provided on the outer surface side of the bag body 102 and through which the band body 11 is inserted.

The bag body 102 is formed by sewing elastic fabric into a rectangular bag-like shape, for example, and the heat buffer material 103 is sealed therein.

Examples of the heat buffer material 103 include resin foam beads and urethane foam, and among others, resin foam beads which easily fit the body of the user are preferable.

The pocket part 104 is made of, for example, rectangular thick fabric, and formed such that three sides on the outer perimeter of the rectangular fabric are stitched to the three sides on the outer perimeter of the bag body 102, and one remaining side of the rectangular fabric is open.

The loop part 105 is made of, for example, thick fabric, and formed such that the two opposite sides on the outer perimeter of the rectangular fabric are stitched to the two sides on the outer perimeter of the bag body 102 so as to form an opening, into which the belt body 11 is inserted, on the opposite side from the opening of the pocket part 104.

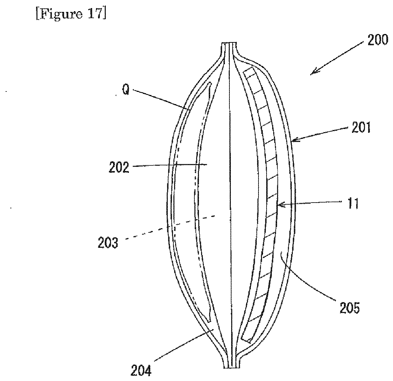

FIG. 17 is an explanatory view illustrating the third embodiment of the support band according to the present invention. Note that the components in FIG. 17 same as the components in FIGS. 16(A) to (C) are identified by the same reference marks.

The support band 200 according to the third embodiment is also provided with the belt body 11 and a bag member 201, and is the same as the support band in the second embodiment, except that the pocket part 104 of the bag member 101 in the second embodiment described with reference to FIG. 16(A) is provided on the side opposite to the loop part 105. It is to be noted that the bag member 201 according to the third embodiment provided with a bag body 202 that accommodates a heat buffer material 203, a pocket part 203, and a loop part 205 can also be obtained by inverting the loop part 105 of the bag member 101 in the second embodiment.

For example, when intending to cool a part of the body, the user wears the support band 200 according to the third embodiment with a commercially available refrigerant Q being put into the pocket part 204 of the bag member 201. Thus, the user can cool a part of the body. In this case, due to the heat buffer material 203 being provided between the refrigerant Q and the band body 11, the refrigerant Q can be placed closer to the user, whereby the lower back of the user can be efficiently cooled.

The bag member 101 in the second embodiment and the bag member 201 in the third embodiment may be made of honeycomb knit formed by three-dimensional knitting.

### <Eleventh wearing example>

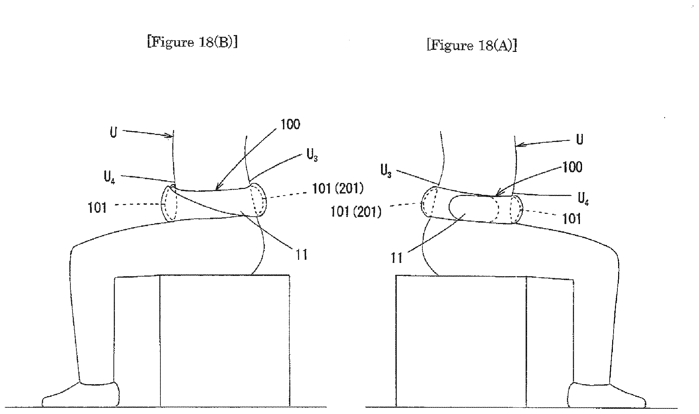

FIG. 18 illustrates an eleventh wearing example for warming or cooling the abdominal region and the lower back region using the support band according to the second or third embodiment, wherein (A) is a view seen from the right side, and (B) is a view seen from the left side.

When the user intends to warm the abdomen and the lower back, commercially available disposable body warmers K (see FIG. 16(C)) are put into the pocket parts 104 of the two bag members 101 of the support band 100 according to the second embodiment, respectively, and the support band 100 is wrapped around the waist of the user U. Thus, the lower back U₃ and the abdomen U₄ can be warmed, while the lower back U₃ is supported. Note that only one bag member 101 may be provided, and either the lower back U₃ or the abdomen U₄ may be warmed.

The support band 100 provided with the bag member 101 and the band body 11 according to the second embodiment is suitable for commute and outdoor work in a cold season, for example. The support band 100 is also preferable for alleviating menstrual cramps of female clerks who may work for many hours in a seated position. In such a case, due to the heat buffer material 103 being provided between the body of the user U and the disposable body warmer K, heat of the disposable body warmer K transmitted to the user U is alleviated, and thus, an occurrence of low temperature injury can be prevented. In addition, the support band 100 allows the use of a relatively inexpensive disposable body warmer K, and thus can prevent frequent use of expensive heating body warmers for alleviating menstrual cramps, thereby reducing economical burdens on the user U. Further, due to the configuration in which the heat buffer material 103 is formed from resin foam beads, if the resin foam beads are collected to the central part of the bag body 102 to increase the thickness, the heat alleviation is increased, and if the resin foam beads are collected to the outer peripheral part of the bag body 102 to reduce the thickness at the central part, the heat alleviation can be reduced. In this way, the temperature of heat transmitted to the user U can be easily adjusted.

Further, if the user needs to cool the lower back for alleviating acute low back pain, the commercially available refrigerants Q (see FIG. 17) are put into the pocket parts 104 of the bag members 201 of the support band 200 according to the third embodiment, respectively, and the support band 200 is wrapped around the waist of the user U. Thus, the lower back U₃ can be supported and cooled simultaneously.

### <Twelfth wearing example>

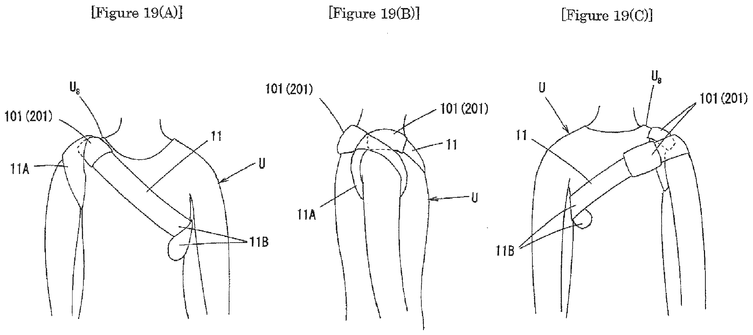

FIG. 19 illustrates a twelfth wearing example for warming or cooling the shoulder using the support band according to the second or third embodiment, wherein (A) is a view seen from front, (B) is a view seen from the right side, and (C) is a view seen from back.

For example, if the user intends to warm the shoulder U₈ for alleviating the stiffness (stiff shoulder), the commercially available disposable body warmers K (see FIG. 16(C)) are put into the pocket parts 104 of the two bag members 101 of the support band 100 according to the second embodiment, respectively, and the support band 100 is wrapped around one of the shoulders of the user U. In this case, the support band 100 can be worn in such a way that the intermediate part of the stretchable part 11A of the band body 11 is wrapped around the shoulder from the underarm, and the hook-and-loop fastener parts 11B on both ends are crossed on the other underarm in a criss-cross manner (cross multiplication) and bonded to each other by surface contact, as illustrated in FIGS. 19(A) to (C).

Alternatively, when a pitcher intends to cool the shoulder U₈ after a baseball game or baseball practice, commercially available refrigerants Q (see FIG. 17) are put into the pocket parts 104 of the two bag members 201 of the support band 200 according to the third embodiment, and the support band 200 is wrapped around one of the shoulders of the user U.

In such cases, the support band may be provided with only one bag member 101.

### <Thirteenth wearing example>

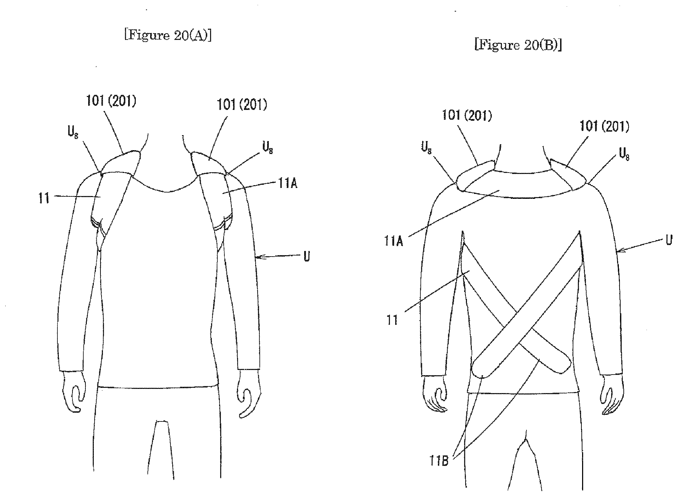

FIG. 20 illustrates a thirteenth wearing example for warming or cooling both shoulders using the support band according to the second or third embodiment, wherein (A) is a view seen from front, and (B) is a view seen from back.

If the user intends to warm both shoulders U₈, commercially available disposable body warmers K (see FIG. 16(C)) are put into the pocket parts 104 of the two bag members 101 of the support band 100 according to the second embodiment, respectively, and the support band 100 is wrapped around the both shoulders of the user U. In this case, as illustrated in FIGS. 20(A) and (B), the support band 100 can be worn in such a way that the intermediate part of the stretchable part 11A of the band body 11 is placed on the back of the neck, and the hook-and-loop fastener parts 11B on both ends are crossed with each other on the back through both underarms and bonded to each other by surface contact.

When the user intends to cool both shoulders U₈, commercially available refrigerants Q (see FIG. 17) are put into the pocket parts 104 of the two bag members 201 of the support band 200 according to the third embodiment, respectively, and the support band 200 is wrapped around the shoulders of the user U.

### <Other wearing examples>

When the user intends to cool the bruised knee in the example of supporting the knee U₁ using the support band 10 described with reference to FIG. 9, the knee can be cooled by the refrigerant using the support band 200 according to the third embodiment.

When the user intends to cool the sprained ankle in the example of supporting the ankle U₆ using the support band 10 described with reference to FIG. 10, the knee can be cooled by the refrigerant using the support band 200 according to the third embodiment.

### (Fourth embodiment)

The first to third embodiments describe the hook-and-loop fastener part 11B formed by providing the hook-shaped fiber surface part 11Ba on one surface and the loop-shaped fiber surface part 11Bb on the other surface of a piece of cloth. However, the present invention is not limited thereto. For example, the hook-and-loop fastener part may be formed such that the hook-shaped fiber surface part is provided on one surface of a piece of cloth, the loop-shaped fiber surface part is provided on one surface of another piece of cloth, and the two pieces of cloth are stitched together with the other surfaces thereof being in contact with each other.

### (Fifth embodiment)

The first to third embodiments describe the support band 10 using a rubber band for only the stretchable part 11A. However, the present invention is not limited thereto. For example, the support band may be configured such that the entire support band is formed from a rubber band, and the hook-shaped fiber surface parts and the loop-shaped fiber surface parts constituting the hook-and-loop fastener parts are continuously or intermittently stitched on both surfaces of the rubber band at both ends.

With this configuration, the elongation rate of the intermediate strip part and the elongation rate of the hook-and-loop fastener part can also be made different from each other. When the hook-shaped fiber surface part and the loop-shaped fiber surface part are intermittently provided in the longitudinal direction, the elongation rate of the hook-and-loop fastener part in the fifth embodiment is smaller than the intermediate strip part, but can be set larger than the hook-and-loop fastener part in the first embodiment.

The embodiments of the present invention described above should be considered in all respects as illustrative and not restrictive of the present invention. The scope of the present invention is not limited to the above description, but the accompanying claims and their equivalents are intended to cover such forms or modifications as would fall within the scope and spirit of the invention.

### DESCRIPTION OF REFERENCE SIGNS

- 11: Band body
- 11A: Intermediate strip part
- 11B: Hook-and-loop fastener part
- 11Ba: Hook-shaped fiber surface part
- 11Bb: Loop-shaped fiber surface part
- 101, 201: Bag member
- 102,202: Bag body
- 103, 203: Heat buffer material
- 104, 204: Pocket part
- 105, 205: Loop part

## Claims

1. A support band comprising a band body which has an intermediate strip part and hook-and-loop fastener parts connected to both ends in a longitudinal direction of the intermediate strip part,
wherein the hook-and-loop fastener parts each have a hook-shaped fiber surface part provided on one surface and a loop-shaped fiber surface part that is provided on the other surface opposite to the one surface and is capable of bonding to the hook-shaped fiber surface part by surface contact,
each of the hook-shaped fiber surface parts and the loop-shaped fiber surface parts is continuously or intermittently provided so as to extend from one end part to the other end part in a longitudinal direction of each of the hook-and-loop fastener parts, and
the band body has a total length allowing the band body to be helically wrapped around a user from the lower abdomen, past the lower back, toward the chest.

2. A support band comprising a band body which has an intermediate strip part and hook-and-loop fastener parts connected to both ends in a longitudinal direction of the intermediate strip part,
wherein the hook-and-loop fastener parts each have a hook-shaped fiber surface part provided on one surface and a loop-shaped fiber surface part that is provided on the other surface opposite to the one surface and is capable of bonding to the hook-shaped fiber surface part by surface contact,
each of the hook-shaped fiber surface parts and the loop-shaped fiber surface parts is continuously or intermittently provided so as to extend from one end part to the other end part in a longitudinal direction of each of the hook-and-loop fastener parts, and
the hook-and-loop fastener parts each have a length allowing the hook-and-loop fastener part to be wrapped around the leg of a user at least once.

3. The support band according to claim 1, wherein the band body also has a total length allowing the band body to be wrapped around the user from the lower abdomen, past the lower back, toward the chest in a double helical manner in reverse directions.

4. The support band according to any one of claims 1 to 3, wherein the intermediate strip part has an elongation rate greater than an elongation rate of each hook-and-loop fastener part.

5. The support band according to any one of claims 1 to 4, wherein the intermediate strip part has an elongation rate of at least 1.1.

6. The support band according to any one of claims 1 to 5, further comprising a bag member attached to the band body in a detachable manner,
wherein the bag member has a bag body, a heat buffer material accommodated in the bag body, a pocket part for accommodating a heating member or for accommodating a heat absorbing member, the pocket part being provided on an outer surface side of the bag body, and a loop part which is provided on the outer surface side of the bag body and through which the band body is inserted.

7. The support band according to claim 6, wherein the heat buffer material is formed from resin foam beads.
